# EUROPEAN PATENT SPECIFICATION

(11) **EP 1 176 988 B1**
(45) Date of publication and mention of the grant of the patent: **24.11.2004**
(21) Application number: 00919400.2
(22) Date of filing: 14.03.2000
(51) Int. Cl.: A61K 51/00, A61M 36/14

(54) **RADIOACTIVE CISPLATIN IN THE TREATMENT OF CANCER**
RADIOAKTIVES CISPLATIN ZUR KREBSBEHANDLUNG.
CISPLATINE RADIOACTIVE UTILISEE DANS LE TRAITEMENT DU CANCER

(43) Date of publication of application: 06.02.2002
(73) Proprietor: Isotope Solutions, Inc., Garden City, NY 11530 (US)
(72) Inventor: ORDER, Stanley, E., Owings Mills, Maryland 21117 (US)
(74) Representative: Shaw, Laurence
(86) International application number: PCT/US2000/006546
(87) International publication number: WO 2000/056369

(56) References cited:
- US-A- 5 484 612
- OWENS S E ET AL: "IN VIVO DISTRIBUTION STUDIES OF RADIOACTIVELY LABELLED PLATINUM COMPLEXES;CIS-DICHLORODIAMMINE PLATINUM(II), CIS-TRANS-DICHLORODIHYDROXY-BIS-(ISOPROPYL AMINE) PLATINUM(IV), CIS-DICHLORO-BIS-CYCLOPROPYLAMINE PLATINUM(II), AND CIS-DIAMMINE 1,1-CYCLOBUTANED" CANCER CHEMOTHERAPY AND PHARMACOLOGY, SPRINGER VERLAG, BERLIN, DE, vol. 14, no. 3, 1985, pages 253-257, XP001099110 ISSN: 0344-5704
- THATCHER N ET AL: "BLOOD CLEARANCE OF THREE RADIOACTIVELY LABELLED PLATINUM COMPLEXES: CIS-DICHLORODIAMMINE PLATINUM II, CIS TRANS-DICHLORODIHYDROXY-BIS-(IS OPROPYLAMINE) PLATINUM IV, AND CIS-DICHLORO-BIS-CYCLOPROPYLAMINE PLATINUM II, IN PATIENTS WITH MALIGNANT DISEASE" CANCER CHEMOTHERAPY AND PHARMACOLOGY, SPRINGER VERLAG, BERLIN, DE, vol. 9, no. 1, 1982, pages 13-16, XP001099115 ISSN: 0344-5704
- BATES ET AL: "THE SYNTHESIS OF 191Pt LABELLED JM216, AN ORALLY ACTIVE PLATINUM ANTI-TUMOR AGENT" APPLIED RADIATION AND ISOTOPES, PERGAMON PRESS LTD., EXETER, GB, vol. 48, no. 4, 1997, pages 469-476, XP002212673 ISSN: 0969-8043
- BAER J ET AL: "MICROSCALE SYNTHESES OF ANTI-TUMOUR PLATINUM COMPOUNDS LABELLED WITH 191PT" INTERNATIONAL JOURNAL OF APPLIED RADIATION AND ISOTOPES, PERGAMON PRESS LTD. OXFORD, GB, vol. 36, no. 3, 1985, pages 181-184, XP001099112
- JACKSON H ET AL: "AN IMPROVED SYNTHETIC PROCEDURE FOR THE PREPARATION OF 195MPT LABELLED ANTI-TUMOUR COMPLEXES" JOURNAL OF LABELLED COMPOUNDS AND RADIOPHARMACEUTICALS, SUSSEX, GB, vol. 29, no. 10, October 1991 (1991-10), pages 1121-1130, XP001097907 ISSN: 0362-4803

## Description

This invention relates to the use of radioactive cisplatin in the treatment of cancer.

In *Cancer Chemother. Pharmacol*. (1982) **9;** pp 13 - 16, Thatcher *et al* determine the blood clearance of, *inter alia,* cisplatin, prepared using ¹⁹¹Pt and ¹⁹³Pt. They suggested that the use of radioactive platinum would allow *in vivo* dynamic imaging of the distribution of platinum compounds in areas of interest.

Bates *et al* (*Appl. Radiat. Isot*. (1997) **48 (4);** pp 469 - 476) disclose a procedure for synthesising a radioactive version (¹⁹¹Pt or ¹⁸⁸Pt) of JM216, an orally active antitumour complex. The radioactive version is said to be useful as a biological tracer.

Baer *et al* (*Int. J. Appl. Radiat. Isot*. (1985) **36 (3);** pp 181 - 184) have disclosed a microscale synthesis of, *inter alia,* radioactive cisplatin (¹⁹¹Pt) for use as a label for the study of animal organ distribution, patient blood clearance, urinary excretion and renal uptake and clearance.

Jackson *et al* (*J. Labelled Compunds & Radiopharmaceuticals* (1991) **XXIX (10)**; pp 1121 - 1130) disclose an improved procedure for preparing, *inter alia,* radioactive (¹⁹⁵Pt) cisplatin.

Hepatocellular cancer that is non-resectable has had a median survival of six months when treated. A variety of chemotherapeutic agents have been given intravenously, with less-than-realistic results. During the course of investigations in hepatocellular cancer, we found that intra-arterial cisplatin at 50 mg/m² caused a 50 percent remission in 28 patients; and, recently, in 67 patients, a 50 percent response. Thus, 95 patients have had a 50 percent remission, with median survival extended one year to 46 months depending on the classification of the tumor. In addition to these findings, the median survival rate for the worst group of hepatomas (AFP+ positive) was one year greater than the six months reported from the other therapeutic regimens.

In order to discern why intra-arterial cisplatin was superior to intravenous, we created 195^{m} cisplatin, and studied the same patient intravenously and intra- arterially. When the drug was given intravenously, less than five percent deposited in the tumor. When the drug was given intra-arterially, 35 to 40 percent of the drug deposited in the tumor, as witnessed by scintigraphic scans. The cisplatin that binds on the hepatoma apparently stays there for a persistent period of time and interrupts DNA metabolism. The use of intravenous cisplatin has had less than a five-percent response rate, and the arterial route is 50 percent or greater.

Having studied the diagnostic distribution of 195^{m} cisplatin, one realized that the possibility of treating with a radioactive cisplatin exists.

We propose a Phase 1 dose escalation in which 10 mCi of radioactivity, approximately 10 mg of the drug, are substituted for the cold in the infusion of the liver. The dose would be escalated as long as there is no significant hepatologic toxicity. The maximum dose we presently give of 50 mg/m² on an average person would be about 70 to 75 mg. The procedure would take the 195^{m} cisplatin and substitute it for the cold, and the patient would get both the cold cisplatin and the radioactive cisplatin. Cisplatin is also a radiation sensitizer; and, as much, the drug should amplify the therapeutic results from the radiolabeling.

The benefit to the patient would be that the non-radioactive form and the radioactive form would still behave as the drug cisplatin, since it is created radioactive by neutron bombardment and not by chemistry alteration or linkage.

Cisplatin has been used diagnostically in a variety of tumors where its direct infusion is part of the pharmacokinetics. Therapy with 195^{m} cisplatin would have us in hepatoma, bladder and brain tumors, but has never been proposed for this use.

A dosage range of approximately 10mCi to 75 mCi or more, depending upon the size of the tumor, may be given in accordance with this invention for all types of solid tumor cancers and not limited to hepatomas.

Without further elaboration, the foregoing so fully illustrates my invention that others may adapt and use the invention accordingly.

## Claims

1. Use of a composition comprising radioactive cisplatin for the manufacture of a drug for treating cancer.

2. Use according to Claim 1, **characterized in that** the amount of cisplatin is about 10mCi to about 75 mCi.

3. Use according to Claim 1, **characterized in that** the composition is administerable intra-arterially.

4. Use according to Claim 1, **characterized in that** the cisplatin is 195^{m} cisplatin.

## Patentansprüche

1. Verwendung einer Zusammensetzung, die Cisplatin enthält, zur Herstellung eines Arzneimittels für die Behandlung von Krebs.

2. Verwendung nach Anspruch 1, **dadurch gekennzeichnet, daß** die Menge des Cisplatins etwa 10 mCi bis etwa 75 mCi beträgt.

3. Verwendung nach Anspruch 1, **dadurch gekennzeichnet, daß** die Zusammensetzung intraarteriell verabreichbar ist.

4. Verwendung nach Anspruch 1, **dadurch gekennzeichnet, daß** das Cisplatin 195^{m}-Cisplatin ist.

## Revendications

1. Utilisation d'une composition comprenant du cisplatine radioactif pour la fabrication d'un médicament pour traiter le cancer.

2. Utilisation selon la revendication 1, **caractérisée en ce que** la quantité de cisplatine est d'environ 10 mCi à environ 75 mCi.

3. Utilisation selon la revendication 1, **caractérisée en ce que** la composition est administrable par voie intra - artérielle.

4. Utilisation selon la revendication 1, **caractérisée en ce que** le cisplatine est le cisplatine 195^{m}.
